# EUROPÄISCHE PATENTSCHRIFT

(11) **EP 0 923 356 B1**
(45) Veröffentlichungstag und Bekanntmachung des Hinweises auf die Patenterteilung: **04.09.2002**
(21) Anmeldenummer: 98934936.0
(22) Anmeldetag: 09.06.1998
(51) Int. Cl.: A61F 5/00

(54) **LAPAROSKOPISCH EINSETZBARES MAGENBAND**
GASTRIC TAPE INSERTABLE BY LAPAROSCOPY
SANGLE GASTRIQUE INSERABLE PAR LAPAROSCOPIE

(30) Priorität: 09.06.1997 DE 19724127; 22.11.1997 DE 19751733
(43) Veröffentlichungstag der Anmeldung: 23.06.1999
(73) Patentinhaber: Pier, Arnold, 52525 Heinsberg (DE)
(72) Erfinder: PIER, Arnold, D-52525 Heinsberg (DE); HUMMEN, Jörg, D-52064 Aachen (DE)
(74) Vertreter: Dallmeyer, Georg, Dipl.-Ing.
(86) Internationale Anmeldenummer: EP9803447
(87) Internationale Veröffentlichungsnummer: WO98056321

(56) Entgegenhaltungen:
- EP-A- 0 611 561
- EP-A- 0 769 282
- WO-A-94/27504
- US-A- 4 632 114
- US-A- 5 074 868

## Beschreibung

Die Erfindung betrifft ein laparoskopisch einsetzbares Magenband zur Umschlingung des Magens zwecks Verkleinerung des Magenvolumens nach dem Oberbegriff des Anspruchs 1.

Derartige Magenbänder haben sich zur Behandlung von krankhafter Fettleibigkeit bewährt. Bei einem bekannten Magenband (WO 94/27504) ist ein Schlingenteil von einem Ballonelement umgeben, das mit einem Fluid dehnbar ist, um den Schlingendurchmesser zu verringern. Das Fluid wird über eine Schlauchleitung von einem Fluidport in das Ballonelement hineingedrückt, wodurch sich der Innenquerschnitt des schlingenförmigen Magenbandes verringert. Der Schlingenteil ist mit einem Verschlußteil zur Fixierung der Schlingenweite versehen.

Der Fluidport wird in der Nähe der Bauchdecke plaziert, um mit Hilfe einer transdermalen Injektion dem Fluidport Fluid zuzuführen oder zu entnehmen und dementsprechend eine Einstellung der Schlingenweite zu ermöglichen.

Bei dem bekannten Magenband besteht das Ballonelement aus einem schlauchförmigen, die Schlinge umgebenden Ballon, der den Nachteil hat, sich nicht nur radial nach innen auszudehnen, sondern auch seitlich, was unerwünscht ist und im übrigen die Zufuhr eines erhöhten Fluidvolumens erfordert. Desweiteren wird für die laparoskopische Anwendung ein großer Trokardurchmesser benötigt. Ein weiterer Nachteil des bekannten Magenbandes besteht darin, daß dessen Lage an dem Magen nicht fixierbar ist. Bei den bekannten Magenbändern ist es häufig notwendig, eine Nachoperation durchzuführen, wenn das Band verrutscht ist. Das Magenband kann auch umklappen, wodurch unbeabsichtigt der Durchlaßquerschnitt des Magens verringert oder ganz geschlossen wird, so daß in jedem Fall eine weitere Operation erforderlich wird. Zur Absicherung der Lage des Magenbandes, ist es lediglich bekannt, die beiden Magenhälften, die von dem Magenband getrennt werden, aneinanderzunähen, wodurch der Magen aus seiner natürlichen Lage entfernt wird.

Der Erfindung liegt die Aufgabe zugrunde, ein mit größerer Sicherheit handhabbares und einfacher herstellbares Magenband zu schaffen.

Zur Lösung dieser Aufgabe dienen die Merkmale des Anspruchs 1. Die Erfindung sieht in vorteilhafter Weise vor, daß beiderseits des Schlingenteils jeweils eine Befestigungseinrichtung mit Perforationen angeordnet ist. Die Befestigungseinrichtung ermöglicht es, ohne die Lage des Magens zu verändern, die Position des Magenbandes an dem Magen zu fixieren. Das Magenband kann dadurch infolge von Magen- oder Körperbewegungen nicht verrutschen. Die seitlichen Befestigungseinrichtungen verhindern in zuverlässiger Weise ein Verrutschen des Magenbandes und sind für Klammergeräte verwendbar, so daß sie die Verkürzung der Operationszeit sowie eine exakte Positionierung des Magenbandes ermöglichen.

Ergänzend ist vorgesehen, daß an dem Fluidport mehrere Befestigungslaschen angeformt sind, die ebenfalls der Lagefixierung des Fluidports in der Nähe der Bauchdecke dienen. Die an den Fluidport angeformten Befestigungslaschen sind dabei ebenfalls so gestaltet, daß sie von einem Klammergerät verklammert werden können, ohne den Fluidport in aufwendiger Weise vernähen zu müssen.

Bei einem Ausführungsbeispiel ist vorgesehen, daß der Schlingenteil auf der Innenseite ein Abdeckteil aus leicht dehnbaren Material aufweist, das gemeinsam mit dem Schlingenteil ausschließlich auf dessen Innenseite ein dehnbares Ballonelement bildet. Das Ballonelement wird demzufolge durch ein auf das Schlingenteil aufsetzbares Abdeckteil aus im Vergleich zum Schlingenteil deutlich dünnerem Material gebildet. Das Abdeckteil kann dabei das Schlingenteil im Querschnitt gesehen U-förmig umschließen, wobei die Seitenteile und die stirnseitigen Enden des Abdeckteils mit dem Schlingenteil verklebt sind. Auf diese Weise kann sich das aus dem Abdeckteil und dem Schlingenteil gebildete Ballonelement nur in Radialrichtung nach innen ausdehnen, ohne daß das Ballonelement gleichzeitig eine seitliche Ausdehnung erfährt. Die Bildung des Ballonelementes mit Hilfe eines Abdeckteils verbilligt den Fertigungsaufwand, wodurch das Magenband erheblich preiswerter produzierbar ist.

Bei einem besonders bevorzugten Ausführungsbeispiel ist das Abdeckteil einstückig mit dem Schlingenteil, wobei der Schlingenteil als schlauchförmiger Hohlkörper gestaltet ist und auf der dem Magen zugewandten Seite eine dünnere Materialstärke aufweist.

Vorzugsweise geht das Abdeckteil auf der Innenseite des Schlingenteils stufenlos in das Verschlußelement über.

Dabei ist auch der Verschlußteil an seiner Innenfläche dem Schlingendurchmesser angepaßt.

Auf diese Weise bildet die Innenseite des Magenbandes im geschlossenen Zustand des Schlingenteils einen kreisförmigen Querschnitt ohne Stufensprünge.

Bei einem bevorzugten Ausführungsbeispiel der Erfindung ist vorgesehen, daß der Schlingenteil und/oder das Abdeckteil aus einem mit einem Röntgenkontrastmittel versehenen Silikongummi besteht. Auf diese Weise ist es möglich, mit Hilfe einer Röntgenaufnahme die Lage des Magenbandes und dessen Innendurchmesser genau zu bestimmen.

Alternativ ist es möglich, in dem Schlingenteil mehrere Röntgenreflektoren vorzusehen.

Beispielsweise bestehen die Röntgenreflektoren aus metallischen Streifen, die quer zu dem Schlingenteil mit gleichem Abstand voneinander verlaufen. Die Röntgenreflektoren ermöglichen eine exakte Lokalisierung des Magenbandes selbst bei einem Röntgenkontrastmittel im Magen.

Die Röntgenreflektoren sind vorzugsweise an dem Abdeckteil angeordnet, so daß sie gleichzeitig den Innendurchmesser der Schlinge anzeigen, der beispielsweise während der Röntgenuntersuchung verändert werden kann.

Der Fluidport wird vorzugsweise an seinem Boden mit einem metallischen Kreisring versehen, wobei der den Fluidport dicht abschließende Silikonstopfen von einem Metallring umfaßt ist. Die Metallringe ermöglichen es ebenfalls bei einer Röntgenaufnahme die Lage des Fluidports unter der Bauchdecke zu detektieren, wobei die Metallringe bei der Positionierung der Injektionsnadel durch die Bauchdecke hindurch behilflich sind.

Der Schlingenteil ist vorzugsweise durch den angespritzten Ösenteil druckdicht verschlossen. Der angespritzte Ösenteil besteht dabei vorzugsweise aus einem härteren Silikonmaterial.

Der Schlingenteil weist vorzugsweise ein inneres längsverlaufendes Stegelement auf, das in den Hohlraum des Ballonelementes hineinragt und den Querschnitt des Hohlraumes verringert.

Dies hat den Vorteil, daß das Fluidvolumen im Ausgangszustand verringert ist, und daß die Profilierung die Steifigkeit des Schlingenteils erhöht. Außerdem wird die Eindrückbarkeit des Magenbandes verringert.

Besonders bevorzugt steht das sich längserstreckende Stegelement von der dem Magen zugewandten Innenseite des Schlingenteils ab.

Auf der Außenseite des Schlingenteils kann ein sich längserstreckender Markierungsstreifen vorgesehen sein. Dieser Markierungsstreifen erleichtert dem Operateur die Identifikation der Außenseite des Magenbandes.

Weitere vorteilhafte Ausgestaltungen des Magenbandes sind den weiteren Unteransprüchen zu entnehmen.

Im folgenden werden unter Bezugnahme auf die Zeichnungen Ausführungsbeispiele der Erfindung näher erläutert.

Es zeigen:
- Fig. 1: ein an einem Magen angelegtes Magenband mit seiner Verbindung zu einem Fluidport,
- Fig. 2: eine Ansicht des Magenbandes im geschlossenen Zustand der Schlinge,
- Fig. 3: eine Seitenansicht des Magenbandes in geöffnetem Zustand,
- Fig. 4: eine Draufsicht auf das Magenband in geöffnetem Zustand,
- Fig. 5: einen Längsschnitt durch das Magenband,
- Fign.6 und 7: einen Querschnitt durch das Magenband,
- Fig. 8: eine Draufsicht auf den Fluidport, und
- Fig. 9: einen Querschnitt durch den Fluidport.
- Fig. 10: eine Seitenansicht eines zweiten Ausführungsbeispiels des Magenbandes,
- Fig. 11: eine Draufsicht auf das zweite Ausführungsbeispiel,
- Fig. 12: einen Schnitt entlang der Linie XII-XII in Fig. 11,
- Fig. 12A und 12B: alternative Querschnittsformen des Schlingenteils,
- Fig. 13: ein Instrument zum Schließen des Magenbandes,
- Fig. 14: eine Seitenansicht des Instrumentes gemäß Fig. 13,
- Fig. 15: den Einsatz des Instrumentes zum Verschließen des Magenbandes, und
- Fig. 16: eine Ansicht in Richtung des Pfeils XVI in Fig. 15.

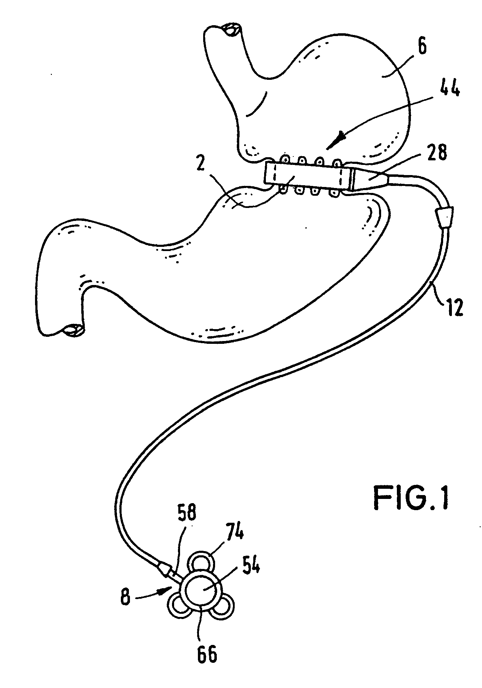

Das in Fig. 1 gezeigte Magenband ist laparoskopisch mit Hilfe eines Trokars in die Bauchhöhle einführbar und kann zur Umschlingung des Magens 6 zwecks Verkleinerung des Magenvolumens verwendet werden. Das Magenband besteht aus einem Schlingenteil 2, in dem ein mit einem Fluiddruck beaufschlagbares Ballonelement 4 enthalten ist. Das Ballonelement 4 ist über eine Schlauchleitung 12 mit einem Fluidport 8 verbunden, der in der Nähe der Bauchdecke plaziert ist. Das Ballonelement 4 ist auf der Innenseite des Schlingenteils 2 angeordnet, so daß das Ballonelement bei Fluidbeaufschlagungen den Schlingeninnendurchmesser verringert. Das Fluid kann mit Hilfe einer transdermalen Injektion in den Fluidport 8 eingespritzt oder entnommen werden. Hierzuweist der Fluidport 8, der aus einer einseitig offenen vorzugsweise zylindrischen Kammer 50 besteht, an dem offenen Ende der Kammer einen Silikonstopfen 54 auf, der bei Perforation mit einer Injektionsnadel selbstdichtend ist. Mit einer Injektionsspritze kann demzufolge durch Einspritzen des Fluids das Ballonelement 4 ausgedehnt werden und damit der Innendurchmesser des Magenbandes vergrößert werden.

Beim Einsetzen des Magenbandes ist die Schlauchleitung 12 zunächst noch nicht mit dem Fluidport 8 verbunden. Zur Erleichterung der Einfädelung der Schlauchleitung 12 in eine Öffnung 34 des Schlingenteils 2 kann das freien Ende der Schlauchleitung 12 zunächst mit einem in den Zeichnungen nicht dargestellten hakenförmigen Endstück versehen sein, das nach der Einführung der Schlauchleitung 12 ein Herausgleiten der Schlauchleitung 12 aus der Öffnung 34 verhindert. Das Ballonelement 4 ist dabei bei aufgesetztem Endstück evakuiert.

Nachdem die Schlinge des Magenbandes zugezogen und arretiert ist, kann das hakenförmige Endstück entfernt werden und die Schlauchleitung 12 mit dem Fluidport verbunden werden.

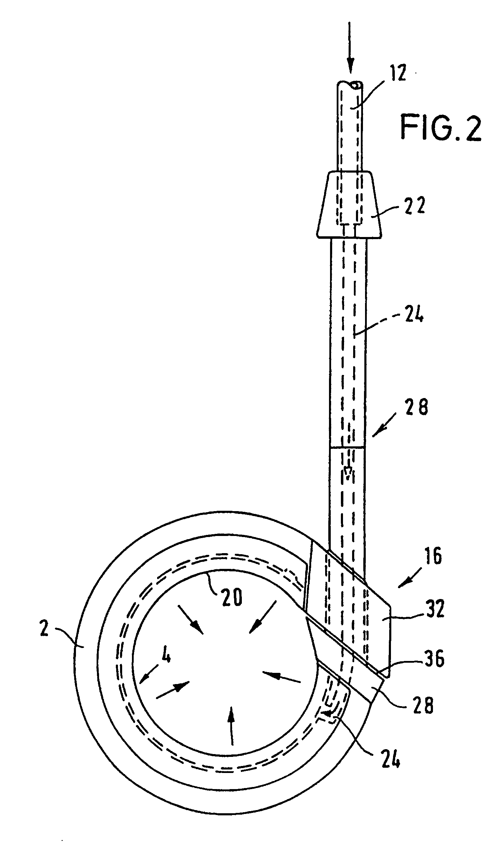

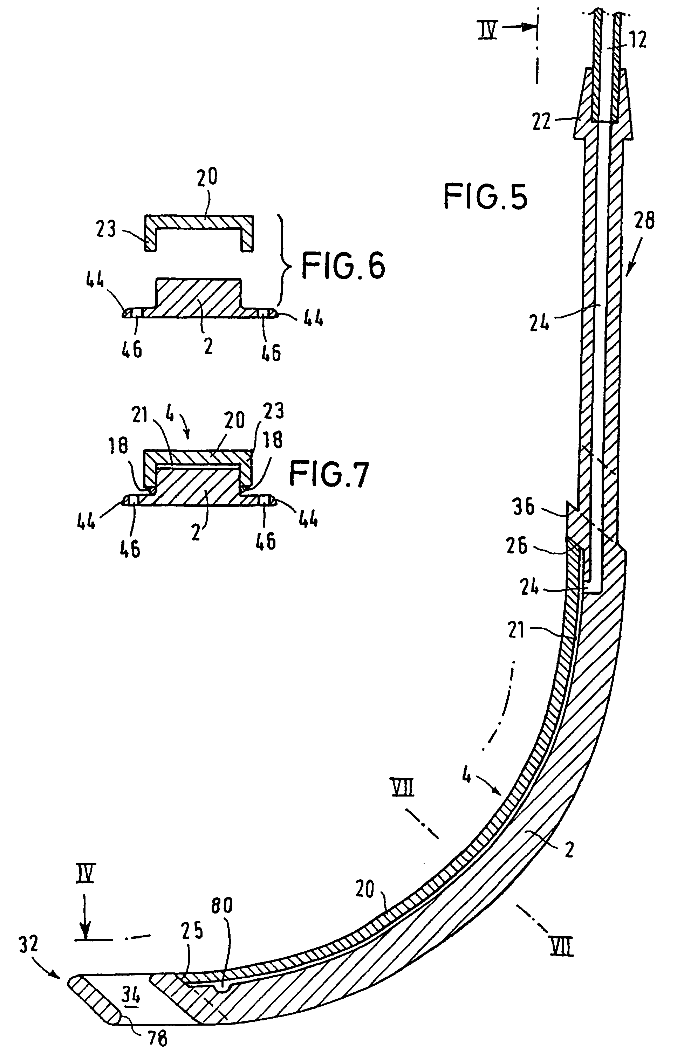

Fig. 2 zeigt den Schlingenteil 2 mit einem das Ballonelement 4 bildenden Abdeckteil 20 aus leicht dehnbarem Material. Das Abdeckteil 20 besteht vorzugsweise aus dem gleichen Material wie der Schlingenteil, und ist im Vergleich zu diesem, wie aus den Fign. 5 bis 7 ersichtlich, erheblich dünner. Als Material wird vorzugsweise "medicalgraded" Silikon mit einer Shore-Härte über 40 verwendet.

Das Ballonelement 4 kann in einfacher Weise durch Verkleben des Abdeckteils 20 mit dem Schlingenteil 2 gebildet werden. Das Abdeckteil 20 umschließt den Schlingenteil 2 unter Bildung eines Hohlraums 21 U-förmig.

Das Abdeckteil 20, das, wie aus Fig. 6 ersichtlich, auf das Schlingenteil 2 aufgesetzt wird, wird an den seitlichen Flanschen 23 und an den Stirnflächen 25,26 mit Hilfe einer Klebewulst 18 dicht mit dem Schlingenteil 2 verbunden. Dabei erstreckt sich das Abdeckteil 20 von dem Ösenteil 32 bis zu dem Laschenteil 28 und geht dabei stufenlos in das Ösenteil 32 an der Stirnfläche 25 bzw. in den Anschlag 36 des Laschenteils 28 an der Stirnfläche 26 über. Auf diese Weise weist der Innendurchmesser des Magenbandes keine Stufen auf. Hierzu ist vorzugsweise auch der radial innere Abschnitt des Laschenteils 28 an die Krümmung des Innendurchmessers angepaßt.

Fig. 2 zeigt das Magenband in geschlossenem Zustand, wobei das Magenband mit Hilfe eines Verschlußteiles 16 in der gezeigten Position arretiert ist.

Der Verschlußteil 16 besteht aus einem Laschenteil 28 an einem Ende des Schlingenteils 2 und einem Ösenteil 32 an dem anderen Ende des Schlingenteils 2. Der Laschenteil 28 wird durch eine Öffnung 34 des Ösenteils 32 hindurchgeführt. Der Ösenteil 32 liegt an einem Anschlag 36 des Laschenteils 28 an und ist in dieser Position durch seitlich gegenüber der Öffnung 34 überstehenden Vorsprüngen 38 des Laschenteils 28 gesichert. Der Ösenteil 32 verschließt die Hohlkammer 21 des Schlingenteils 2 druckdicht, wobei der Ösenteil 32 vorzugsweise an den Schlingenteil 2 angespritzt ist. Der Verschlußteil 16 aus dem Ösenteil 32 und dem Laschenteil 28 besteht vorzugsweise aus einem Silikonmaterial größerer Härte von ca. Shore 60.

Dieses Ausführungsbeispiel zeigt lediglich einen Verschlußteil 16, der nur eine Rastposition zuläßt. Durch Anordnung mehrerer Vorsprünge 38 an dem Laschenteil sind jedoch unterschiedliche Innendurchmesser des Magenbandes einstellbar.

Der Laschenteil 28 weist einen Fluidkanal 24 auf, der von einem Anschlußteil 22 für die Schlauchleitung 12 an dem freien Ende des Laschenteils 28 bis in den Schlingenteil 2 hineinreicht. Die Fluidleitung 24 mündet in den Hohlraum 21 des Ballonelementes 4, so daß sich das Ballonelement 4 ausdehnen kann, wenn das Fluidvolumen durch Zuspritzen des Fluids in den Fluidport 8 erhöht wird. Dadurch verringert sich der Innendurchmesser des Magenbandes, wie in Fig. 2 durch Pfeile angedeutet, ohne daß sich der Außendurchmesser des Magenbandes verändert.

Umgekehrt kann durch Entnahme von Fluid aus dem Fluidport 8 mit Hilfe der Injektionsspritze der Innendurchmesser verringert werden, bis das Abdeckteil 20 bzw. das Ballonelement 4 sich in der in Fig. 5 gezeigten entspannten-Position befindet.

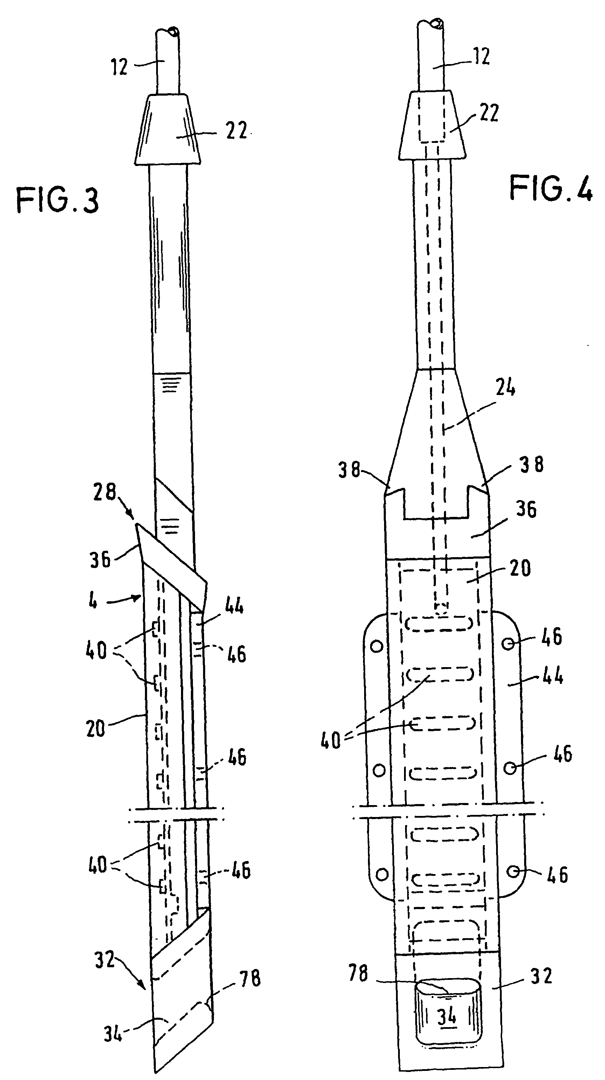

Die Fign. 3 und 4 zeigen seitlich an dem Schlingenteil 2 angeordnete Befestigungslaschen 44, die mit Perforationen 46 versehen sind. Diese Befestigungslaschen 44 ermöglichen es, das Magenband in einer bestimmten Position am Magen zu fixieren, ohne daß das Magenband aufgrund von Magen- oder Körperbewegungen nachträglich verrutschen kann. Die Perforationen 46 können dazu benutzt werden, das Magenband mit dem Magen 6 zu vernähen oder zu verklammern. Die exakte Fixierung des Magenbandes ist sehr vorteilhaft, weil ein Verrutschen oder ein Umklappen des Magenbandes in der Regel eine nachträgliche Operation erforderlich macht. Die Befestigungslaschen 44 sind in hohem Maße (bis ca. 600 %) dehnbar.

Wie aus den Fign. 3 und 4 ersichtlich, können an dem Abdeckteil 20 Röntgenreflektoren 40 aus metallischen Streifen angeordnet sein, die vorzugsweise einen gleichen Abstand voneinander haben. Alternativ besteht der Schlingenteil 2 und/oder Abdeckteil 20 aus einem Silikongummi, der mit einem Röntgenkontrastmittel, z.B. Bariumsulfat, versetzt ist.

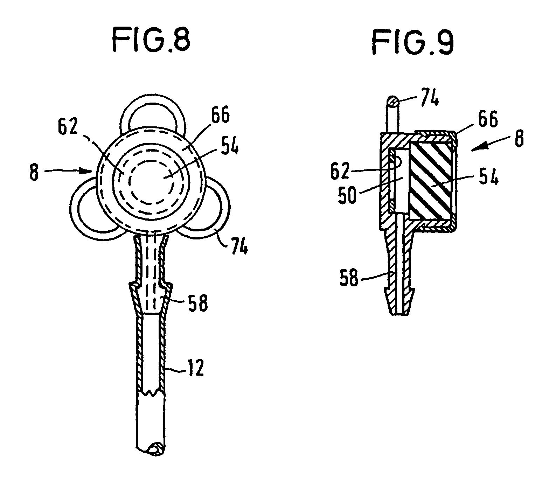

Die Fign. 8 und 9 zeigen den Fluidport 8 in einer Draufsicht bzw. im Querschnitt.

Der Fluidport 8 weist eine vorzugsweise zylindrische einseitig offene Kammer 50 auf, die von einem selbstdichtenden Silikonstopfen 54 verschlossen ist. Ein Metallring 66 faßt den Silikonstopfen 54 ein und sichert ihn an dem Gehäuse des Fluidports 8.

An dem Gehäuse aus Polycarbonat sind drei Befestigungslaschen 74 angeformt, die wie bereits ausgeführt, zum Fixieren des Fluidports 8 durch Vernähen oder Verklammern benötigt werden. Von dem Fluidport 8 steht seitlich ein Schlauchanschluß 58 ab, der die Kammer 50 mit der Schlauchleitung 12 verbindet. Der Schlauch 12 kann auf den Schlauchanschluß 58 irreversibel übergestülpt werden.

Am Boden der Kammer 50 ist eine Ringscheibe 62 angeordnet, die in Verbindung mit dem Metallring 66 eine genaue Ortung des Fluidports 8 auf einem Röntgenbildschirm ermöglicht.

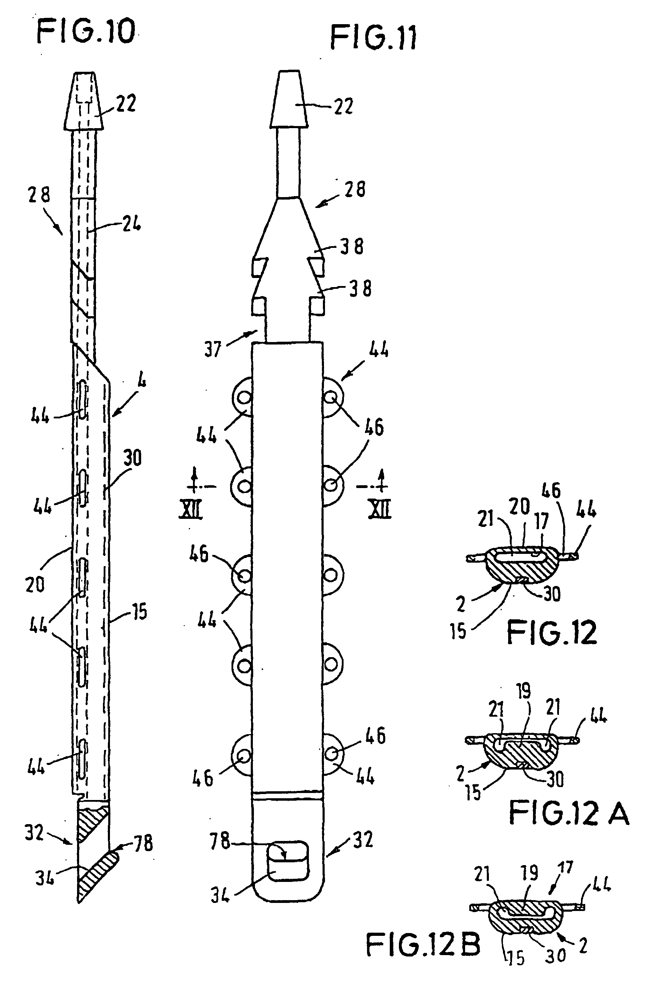

Das Ausführungsbeispiel der Fig. 10 unterscheidet sich von dem zuvor beschriebenen Ausführungsbeispiel in der Querschnittsform, wie am besten aus Fig. 12 ersichtlich ist, wobei das Abdeckteil 20 in den Schlingenteil 2 integriert ist, sowie in dem Laschenteil 28, das zwei Vorsprünge 38 aufweist und somit eine verstellbare Schlingenweite ermöglicht. Selbstverständlich können auch mehr als zwei Vorsprünge 38, die bestimmte Rastpositionen definieren, vorhanden sein. Die Breite des Zwischenstücks 37 zwischen den Vorsprüngen 38 des Laschenteils 28 und dem Ballonelement 4 ist der Öffnungsweite der Öffnung 34 des Ösenteils 32 angepaßt.

Die Befestigungseinrichtung 44 besteht aus mehreren mit Perforationen 46 versehenen Laschen, die beiderseits an den Seitenflächen des Schlingenteils 2 an dem dem Magen 6 zugewandten Ende der Seitenfläche angeformt sind. Die Laschen können mit Hilfe der Perforationen 46 mit der Magenoberfläche vernäht oder verklammert werden. Der Abstand der Perforationen 46 in den Laschen kann dabei an die verwendeten Klammern angepaßt sein. Das Ausführungsbeispiel der Fign. 10 bis 12 kann im übrigen alle weiteren in Verbindung mit dem ersten Ausführungsbeispiel genannten Merkmale aufweisen.

Fig. 12A und 12B zeigen bevorzugte Querschnittsformen des Schlingenteils 2. Ein von dem Abdeckteil 20 oder dem Schlingenteil 2 nach innen abstehendes, sich längserstrekkendes Stegelement 19 verkleinert das Kammervolumen des Hohlraums 21. Dies hat im Hinblick auf die als Fluid verwendete Kochsalzlösung den Vorteil der Volumenminimierung im Ausgangszustand. Außerdem vergrößert der Steg die Stabilität und Steifigkeit des Schlingenteils 2 ohne die Dehnbarkeit des Ballonelementes 4 zu beeinträchtigen. Auf der Außenseite des Schlingenteils 2 kann ein sich längserstreckender Markierungsstreifen 30 in den Schlingenteil 2 eingelassen sein, der sich farblich von dem Verschlußteil 2 absetzt und dem Operateur hilft, die Außenseite des Magenbandes leichter zu identifizieren.

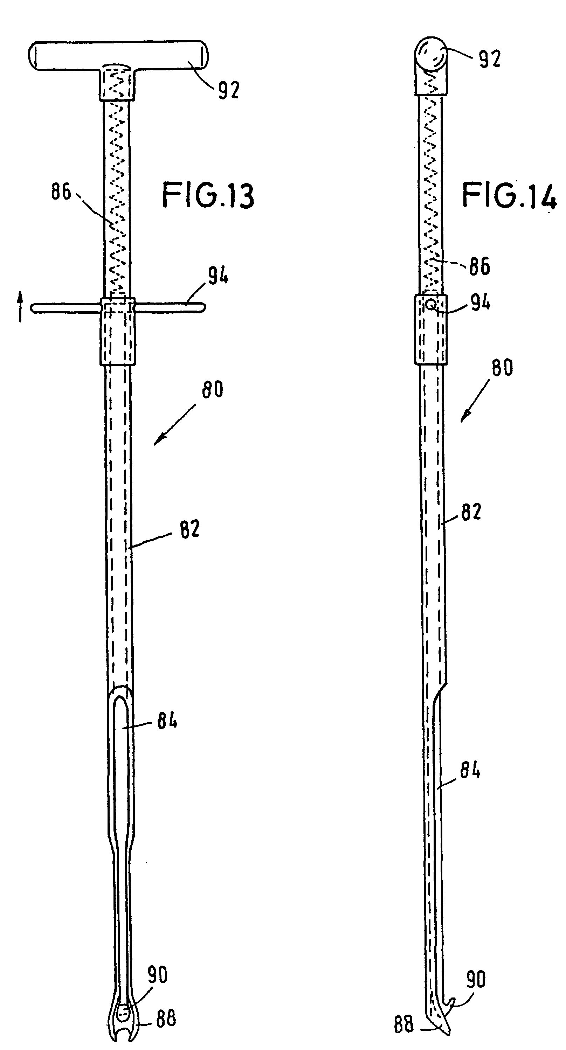

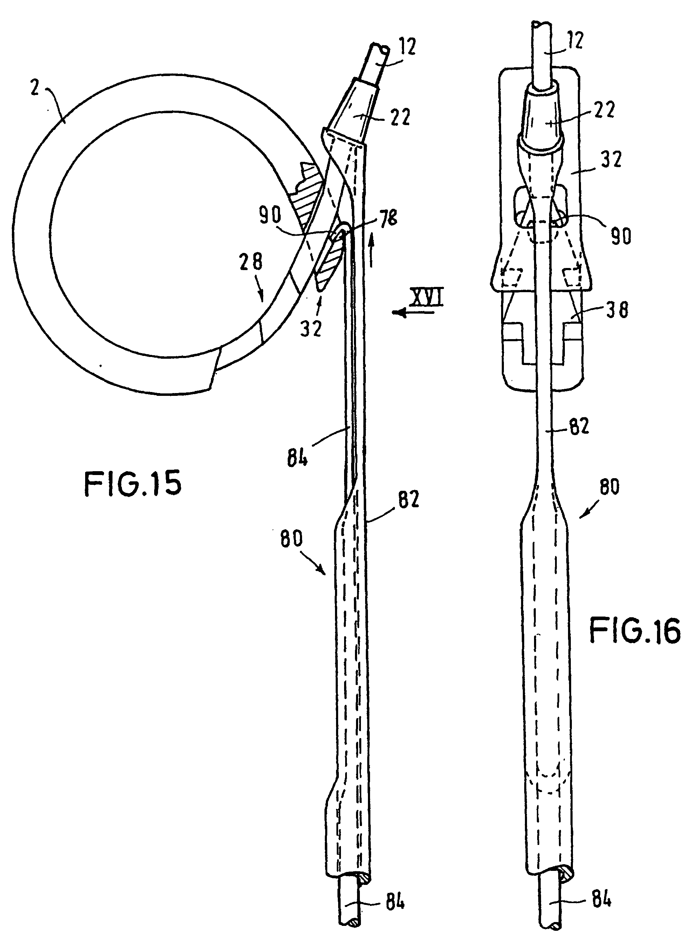

Die Fign. 13 und 14 zeigen ein laparoskopisches Instrument 80, mit dessen Hilfe die Magenschlinge, wie in den Fign. 15 und 16 gezeigt, zugezogen werden kann.

Das laparoskopische Werkzeug 80 besteht aus einem ersten Rohrteil 82, das ein in dem Rohrteil 82 axial verschiebbares Stangenteil 84 aufnimmt. Das Stangenteil 84 ist gegen das Rohrteil 82 mit einer Druckfeder 46 derart vorgespannt, daß das Stangenteil 84 in seiner Ruheposition zum distalen Ende in eine Anschlagposition gebracht ist.

Das Rohrteil 82 weist an seinem distalen Ende ein gabelförmiges Aufnahmeteil 88 für das Anschlußteil 22 des Laschenteils 28 auf.

Das Stangenteil 84 weist an seinem distalen Ende einen Haken 90 auf, der an dem Ösenteil 32 an der Kante 78 angreift.

Die Anwendung des Instrumentes 80 geht aus den Fign. 15 und 16 hervor. Durch Ziehen an dem Handgriff 94 kann das Stangenteil 84, das mit dem Haken 90 an der Kante 78 des Ösenteils angreift, zurückgezogen werden, so daß der Ösenteil 32 über den ersten oder über beide Vorsprünge 38 gezogen werden kann, wodurch die Schlingenweite des Schlingenteils 2 festgelegt ist. Die hintere Kante des Anschlußteils 22 dient dabei als Abstützfläche für das Aufnahmeteil 88 des Instrumentes 80. Die Kante 78 kann relativ zur Außenseite 15 des Schlingenteils 2 wie aus Fig. 10 ersichtlich vorstehen.

Das Instrument 80 kann eingesetzt werden, nachdem der Laschenteil 28 mit dem Anschlußteil 22 durch die Öffnung 34 des Ösenteils 32 hindurchgesteckt ist. Das konische Anschlagteil 22 dient mit seiner hinteren Anschlagfläche dabei in vorteilhafter Weise als vorläufige Arretierung des Laschenteils 28 in der Öffnung 34.

## Patentansprüche

1. Laparoskopisch einsetzbares Magenband zur Umschlingung des Magens (6) zwecks Verkleinerung des Magenquerschnitts
- mit einem Schlingenteil (2), das ein mit Hilfe eines Fluides dehnbares Ballonelement (4) aufweist,
- mit einem Fluidport (8), der über eine Schlauchleitung (12) mit dem Ballonelement (4) des Schlingenteils verbunden ist,
- mit einem Verschlußteil (16) zur Fixierung der Schlingenweite des Schlingenteils (2),
- wobei der Fluidport (8) in der Nähe der Bauchdecke plaziert ist, um zur Einstellung der Schlingenweite durch Füllen oder Entleeren des Ballonselementes (4) mit Fluid dem Fluidport (8) mittels einer transdermalen Injektion Fluid zuzuführen oder zu entnehmen,
**dadurch gekennzeichnet,**
**daß** der Schlingenteil (2) an beiden Seiten jeweils eine Befestigungseinrichtung (44) mit Perforationen (46) aufweist.

2. Magenband nach Anspruch 1, **dadurch gekennzeichnet, daß** an dem Fluidport (8) mehrere Befestigungslaschen (74) angeformt sind.

3. Magenband nach Anspruch 1 oder 2, **dadurch gekennzeichnet, daß** der Schlingenteil (2) als schlauchförmiger Hohlkörper gestaltet ist und auf der dem Magen (6) zugewandten Seite eine im Querschnitt dünnere Materialstärke aufweist.

4. Magenband nach einem der Ansprüche 1 oder 2, **dadurch gekennzeichnet, daß** der Schlingenteil (2) auf der Innenseite ein Abdeckteil (20) aus leicht dehnbarem Material aufweist, das gemeinsam mit dem Schlingenteil (2) ausschließlich auf dessen Innenseite ein dehnbares Ballonelement (4) bildet.

5. Magenband nach Anspruch 4, **dadurch gekennzeichnet, daß** das Abdeckteil einstückig mit dem Schlingenteil (2) ist.

6. Magenband nach einem der Ansprüche 1 bis 5, **dadurch gekennzeichnet, daß** der Verschlußteil (16) des Schlingenteils (2) aus einem die Fluidleitung (24) enthaltenden Laschenteil (28) und einem Ösenteil (32) zum Hindurchstecken des Laschenteils (28) besteht, wobei der Laschenteil (28) in dem Ösenteil (32) in einer oder mehreren Positionen arretierbar ist.

7. Magenband nach einem der Ansprüche 1 bis 5, **dadurch gekennzeichnet, daß** der Verschlußteil (16) des Schlingenteils (2) mit einem Anschlußteil (22) am freien Ende des Laschenteils (28) vorläufig arretierbar ist.

8. Magenband nach einem der Ansprüche 6 oder 7, **dadurch gekennzeichnet, daß** der Laschenteil (28) einen Anschlag (36) für das Ösenteil (32) aufweist.

9. Magenband nach einem der Ansprüche 1 bis 8, **dadurch gekennzeichnet, daß** die Innenseite des Schlingenteils (2) im geschlossenen Zustand der Schlinge einen kreisförmigen Querschnitt ohne Stufensprünge bildet.

10. Magenband nach einem der Ansprüche 1 bis 9, **dadurch gekennzeichnet, daß** der Schlingenteil (2) und/oder das Abdeckteil (20) aus einem mit einem Röntgenkontrastmittel versehenen Silikongummi besteht.

11. Magenband nach einem der Ansprüche 1 bis 7, **dadurch gekennzeichnet, daß** in dem Schlingenteil (2) mehrere Röntgenreflektoren (40) angeordnet sind.

12. Magenband nach Anspruch 11, **dadurch gekennzeichnet, daß** die Röntgenreflektoren (40) aus metallischen Streifen bestehen, die quer zu dem Schlingenteil (2) mit gleichem Abstand voneinander verlaufen.

13. Magenband nach einem der Ansprüche 11 oder 12, **dadurch gekennzeichnet, daß** die Röntgenreflektoren (40) an dem Abdeckteil (20) angeordnet sind.

14. Magenband nach einem der Ansprüche 1 bis 13, **dadurch gekennzeichnet, daß** der Fluidport (8) aus einer einseitig offenen vorzugsweise zylindrischen Kammer (50) besteht, die an dem offenen Ende mit einem bei Perforation mit einer Injektionsnadel selbstdichtenden Silikonstopfen (54) dicht verschlossen ist und die seitlich einen Schlauchanschluß (58) für die Schlauchleitung (12) zu dem Schlingenteil (2) aufweist.

15. Magenband nach Anspruch 14, **dadurch gekennzeichnet, daß** in dem Boden der zylindrischen Kammer (50) ein metallischer Kreisring (62) angeordnet ist.

16. Magenband nach Anspruch 14 oder 15, **dadurch gekennzeichnet, daß** das offene Ende der zylindrischen Kammer (50) von einem den Silikonstopfen (54) haltenden Metallring (66) umfaßt ist.

17. Magenband nach einem der Ansprüche 6 bis 16, **dadurch gekennzeichnet, daß** die Öffnung des Ösenteils (32) eine Kante (78) für den Angriff eines Instrumentes (80) zum Zuziehen der Schlinge aufweist.

18. Magenband nach einem der Ansprüche 3 bis 17, **dadurch gekennzeichnet, daß** der Schlingenteil (2) als schlauchförmiger Körper an einem Ende durch den angespritzten Ösenteil (32) druckdicht verschlossen ist.

19. Magenband nach einem der Ansprüche 1 bis 18, **dadurch gekennzeichnet, daß** der Schlingenteil (2) ein in dem Hohlraum (21) längsverlaufendes Stegelement (19) aufweist, das den Querschnitt des Hohlraums (21) des Ballonelementes (4) verringert.

20. Magenband nach einem der Ansprüche 1 bis 19, **dadurch gekennzeichnet, daß** auf der Außenseite (15) des Schlingenteils (2) ein sich längserstreckender Markierungsstreifen (30) angeordnet ist.

## Claims

1. A gastric band adapted for laparoscopic placement, provided to encircle the stomach (6) for reducing the cross section of the stomach, comprising
- a loop portion (2) comprising a balloon element (4) to be expanded by use of a fluid,
- a fluid port (8) connected to the balloon element (4) of the loop portion via a hose conduit (12),
- a locking member (16) for fixing the loop width of the loop portion (2),
- the fluid port (8) being arranged adjacent the abdominal wall to supply fluid to the fluid port (8) or remove fluid from the fluid port (8) by a transdermal injection for thus adjusting the loop width by filling the balloon element (4) with fluid or discharging fluid from the balloon element (4),
**characterized in**
**that** the loop portion (2) is on both sides provided with a respective attachment device (44) having perforations (46) formed therein.

2. The gastric band according to claim 1, **characterized in that** the fluid port (8) has a plurality of attachment flaps (74) integrally formed thereto.

3. The gastric band according to claim 1 or 2, **characterized in that** the loop portion (2) is provided as a tubular hollow body whose material, when viewed in cross section, is of a reduced thickness on the side facing towards the stomach (6).

4. The gastric band according to claim 1 or 2, **characterized in that** the loop portion (2) has its inner side provided with a cover member (20) made from an easily expanded material which together with the loop portion (2) forms an expansible balloon element (4) exclusively on the inner side of the loop portion (2).

5. The gastric band according to claim 4, **characterized in that** the cover member is integral with the loop portion (2).

6. The gastric band according to any one of claims 1 to 5, **characterized in that** the locking member (16) of the loop portion (2) comprises a flap portion (28) including the fluid channel (24), and an eye portion (32) for inserting the flap portion (28) therethrough, locking of the flap portion (28) in the eye portion (32) being possible in one or a plurality of positions.

7. The gastric band according to any one of claims 1 to 5, **characterized in that** the locking member (16) of the loop portion (2) can be temporarily locked to a connecting member (22) provided on the free end of the flap portion (28).

8. The gastric band according to claim 6 or 7, **characterized in that** the flap portion (28) comprises an abutment portion (36) for the eye portion (32).

9. The gastric band according to any one of claims 1 to 8, **characterized in that**, in the closed condition of the loop, the inner side of the loop portion (2) forms a circular cross section without protruding steps.

10. The gastric band according to any one of claims 1 to 9, **characterized in that** the loop portion (2) and/or the cover member (20) are made from a silicone rubber provided with a radiographic contrast medium.

11. The gastric band according to any one of claims 1 to 7, **characterized in that** a plurality of x-ray reflectors (40) are provided in the loop portion (2).

12. The gastric band according to claim 16, **characterized in that** the x-ray reflectors (40) consist of metallic stripes extending transversely to the loop portion (2) at equal distances from each other.

13. The gastric band according to claim 11 or 12, **characterized in that** the x-ray reflectors (40) are arranged on the cover member (20).

14. The gastric band according to any one of claims 1 to 13, **characterized in that** the fluid port (8) comprises a preferably cylindrical chamber (50) being open on one end, that the chamber (50) has its open end tightly closed by a silicone plug (54) being self-sealing when perforated by an injection needie, and the chamber (50) is provided with a lateral hose connector (58) connecting the hose conduit (12) to the loop portion (2).

15. The gastric band according to claim 14, **characterized in that** a metallic circular ring (62) is arranged on the bottom of the cylindrical chamber (50).

16. The gastric band according to claim 14 or 15, **characterized in that** the open end of the cylindrical chamber (50) is enclosed by a metal ring (66) holding the silicone plug (54).

17. The gastric band according to any one of claims 6 to 16, **characterized in that** the opening of the eye portion (32) comprises an edge (78) to be engaged by an instrument (80) for tightening the loop into a closed position.

18. The gastric band according to any one of claims 3 to 17, **characterized in that** the loop portion (2) formed as a tubular body has one of its ends closed in a pressure-tight manner by the eye portion (32) integrally molded thereto.

19. The gastric band according to any one of claims 1 to 18, **characterized in that** the loop portion (2) is provided with a rib element (19) extending longitudinally in the cavity (21) and reducing the cross section of the cavity (21) of the balloon element (4).

20. The gastric band according to any one of claims 1 to 19, **characterized in that** the outer side (15) of the loop portion (2) is provided with a longitudinal marker strip (30).

## Revendications

1. Sangle gastrique, insérable par laparoscopie, pour enserrer l'estomac (6) en vue de réduire la section de l'estomac comprenant :
- une partie de boucle (2) qui comporte un élément de ballon (4) dilatable à l'aide d'un fluide,
- un orifice (8) pour fluide qui est relié par un conduit (12) en tuyau souple à l'élément de ballon (4) de la partie de boucle ,
- une partie de fermeture (16) pour fixer la taille de boucle de la partie de boucle (2),
l'orifice (8) pour fluide étant placé à proximité de la paroi abdominale pour le réglage de la taille de la partie de boucle par remplissage ou vidage de l'élément de ballon (4) avec du fluide au niveau de l'orifice (8), au moyen d'une injection transdermique pour amener ou retirer le fluide,
**caractérisée en ce que** la partie de boucle (2) comporte sur ses deux côtés respectivement un dispositif de fixation (44) muni de perforations (46).

2. Sangle gastrique selon la revendication 1, **caractérisée en ce que** plusieurs attaches de fixation (74) sont formées au niveau de l'orifice (8) pour fluide.

3. Sangle gastrique selon la revendication 1 ou 2, **caractérisée en ce que** la partie de boucle (2) est réalisée sous forme d'un corps creux de type tuyau souple et présente en coupe transversale une épaisseur de matière plus mince du côté dirigé vers l'estomac (6).

4. Sangle gastrique selon l'une des revendications 1 ou 2, **caractérisée en ce que** la partie de boucle (2) comporte du côté intérieur un revêtement (20) en matériau facilement extensible qui forme conjointement avec la partie de boucle (2), exclusivement du côté intérieur de celle-ci, un élément de ballon (4) dilatable.

5. Sangle gastrique selon la revendication 4, **caractérisée en ce que** le revêtement est d'une seule pièce avec la partie de boucle (2).

6. Sangle gastrique selon l'une des revendications 1 à 5, **caractérisée en ce que** la partie de fermeture (16) de la partie de boucle (2) se compose d'une attache (28) comprenant la conduite de fluide (24), et d'un oeillet (32) pour le passage de l'attache (28), l'attache (28) pouvant être arrêtée dans l'oeillet (32) en une ou plusieurs positions.

7. Sangle gastrique selon l'une des revendications 1 à 5, **caractérisée en ce que** la partie de fermeture (16) de la partie de boucle (2) peut être arrêtée provisoirement avec un raccord (22) à l'extrémité libre de l'attache (28).

8. Sangle gastrique selon l'une des revendications 6 ou 7, **caractérisée en ce que** l'attache (28) comporte une butée (36) pour l'oeillet (32).

9. Sangle gastrique selon l'une des revendications 1 à 8, **caractérisée en ce que** le côté intérieur de la partie de boucle (2), dans l'état fermé de la boucle, forme une section circulaire sans saillie en gradin.

10. Sangle gastrique selon l'une des revendications 1 à 9, **caractérisée en ce que** la partie de boucle (2) et/ou le revêtement (20) se compose d'un caoutchouc silicone pourvu d'un agent contrastant pour les rayons X.

11. Sangle gastrique selon l'une des revendications 1 à 7, **caractérisée en ce que** plusieurs réflecteurs de rayons X (40) sont disposés dans la partie de boucle (2).

12. Sangle gastrique selon la revendication 11, **caractérisée en ce que** les réflecteurs de rayons X (40) se composent de bandes métalliques qui s'étendent transversalement à la partie de boucle (2), à égale distance les unes des autres.

13. Sangle gastrique selon l'une des revendications 11 ou 12, **caractérisée en ce que** les réflecteurs de rayons X (40) sont disposés au niveau du revêtement (20).

14. Sangle gastrique selon l'une des revendications 1 à 13, **caractérisée en ce que** l'orifice (8) pour fluide se compose d'une chambre (50) de préférence cylindrique, ouverte d'un côté, qui est fermée de façon étanche à l'extrémité ouverte par un bouchon de silicone (54) auto-étanchéifiant lors d'une perforation avec une aiguille d'injection et qui comporte latéralement un raccord de tuyau (58) pour la conduite (12) en tuyau souple allant à la partie de boucle (2).

15. Sangle gastrique selon la revendication 14, **caractérisée en ce qu'**une couronne métallique (62) est disposée au fond de la chambre cylindrique (50).

16. Sangle gastrique selon la revendication 14 ou 15, **caractérisée en ce que** l'extrémité ouverte de la chambre cylindrique (50) est entourée par une bague métallique (66) maintenant le bouchon de silicone (54).

17. Sangle gastrique selon l'une des revendications 6 à 16, **caractérisée en ce que** l'ouverture de l'oeillet (32) présente un bord (78) pour l'application d'un instrument (80) en vue de serrer la boucle.

18. Sangle gastrique selon l'une des revendications 3 à 17, **caractérisée en ce que** la partie de boucle (2), en tant que corps creux de type tuyau souple, est fermée de façon étanche à la pression à une extrémité par l'oeillet (32) moulé par injection.

19. Sangle gastrique selon l'une des revendications 1 à 18, **caractérisée en ce que** la partie de boucle (2) comporte un élément de nervure (19), s'étendant longitudinalement dans l'espace creux (21), qui réduit la section de l'espace creux (21) de l'élément de ballon (4).

20. Sangle gastrique selon l'une des revendications 1 à 19, **caractérisée en ce qu'**une bande de repérage (30) s'étendant longitudinalement est disposée sur le côté extérieur (15) de la partie de boucle (2).
